# EUROPEAN PATENT APPLICATION

(11) **EP 1 449 818 A1**
(43) Date of publication of application: **25.08.2004**
(21) Application number: 02777883.6
(22) Date of filing: 18.10.2002
(51) Int. Cl.: C04B 38/00, C04B 35/00, A61L 27/12, A61L 27/56, A61F 2/28

(54) **POROUS ARTICLE OF SINTERED CALCIUM PHOSPHATE, PROCESS FOR PRODUCING THE SAME AND ARTIFICIAL BONE AND HISTOMORPHOLOGICAL SCAFFOLD USING THE SAME**

(30) Priority: 21.10.2001 JP 2001358528
(71) Applicant: National Institute of Advanced Industrial Science and Technology, Tokyo 100-8921 (JP)
(72) Inventor: ITO, Atsuo, Tsukuba-shi, Ibaraki 305-0035 (JP); SAKURAI, Tokoha, Tsukuba-shi, Ibaraki 305-0031 (JP); SOGO, Yu, Tsukuba-shi, Ibaraki 305-0044 (JP); IKEUCHI, Masako, Ikoma-shi, Nara 630-0222 (JP); OHGUSHI, Hajime, Kashihara-shi, Nara 634-0006 (JP)
(74) Representative: Maschio, Antonio, Dr.
(86) International application number: PCT/JP2002/010829
(87) International publication number: WO 2003/035576

(57) **Abstract**

The present invention provides porous material of calcium phosphate of high strength whose open pores penetrate the porous body and have a size of 70 µm or more, preferably 100 µm or more, and are arranged in a three-dimensional network, whose porosity is sufficiently high for blood vessels to invade and perforate itself or for cells to infiltrate itself, whose chemical composition, in particular, Ca/P molar ratio can be freely changed within the range of 0.75 to 2.1, to which elements important for facilitating osteogenesis and producing resorbable effect can be added, and whose phase composition can be relatively easily changed. The invention is porous sintered compact of calcium phosphate which has artificially formed, penetrated open pores 70 µm to 4 mm in diameter, whose porosity is from 20% to 80%, and whose chief ingredient is calcium phosphate having a Ca/P molar ratio of from 0.75 to 2.1.

## Description

### Technical Field

The present invention relates to porous ceramics of calcium phosphate. The porous ceramics of calcium phosphate obtained in accordance with this invention is used as substitute materials for tissue of living bodies, tissue engineering scaffold and a drug carrier for DDS, all of which are required to be biocompatible.

### Background Art

Conventional porous ceramics of calcium phosphate include: for example, porous ceramics which are produced by mixing a resin or organic matter with calcium phosphate raw powder, forming the mixture into compact, and firing the compact so that the portions of the sintered compact from which the resin or organic matter has been removed provide pores (JP Patent Publication (Kokoku) Nos. 2-54303 B (1990), 7-88175 B (1995), 8-48583 B (1996), and many others); which are produced by pouring a slurry to which a foaming agent has been added or a slurry in the foaming state into a mold, drying the poured slurry, and sintering the dried slurry so that the resultant air bubbles provide pores (JP Patent Publication (Kokai) No. 5-270945 A (1993)); which are produced by pouring a slurry to which a foaming agent and a heat-shrinkable resin are added into a mold, drying the poured slurry, and sintering the dried slurry so that the resultant air bubbles provide pores (JP Patent Publication (Kokai) No. 2001-206787 A); which are produced by filling calcium phosphate dense particles into a compacting die, adding a slip formed of a mixture of metaphosphate, an organic binder and a solvent to the compacting die, and sintering the particles together with the slip so that the solvent and the organic binder are removed to provide pores (JP Patent No. 3096930); which are produced by sintering acicular calcium phosphate, set calcium phosphate hydrate or spherical calcium phosphate so that gaps among particles provide pores (JP Patent Publication (Kokai) Nos. 10-259012 A (1998), 9-309775 A (1997), 6-63119 A (1994), 11-322458 A (1999)); which are produced by laminating perforated green sheets of calcium phosphate and sintering the same so that pores are formed (JP Patent Publication (Kokai) No. 11-178913 A (1999); which are produced by sintering calcium phosphate while applying high voltage pulse (JP Patent Publication (Kokai) No. 11-35379 A (1999)); which are produced by integrating noodle-like extrudates of apatite and sintering the integrated extrudates (JP Patent Publication (Kokai) No. 10-245278 A (1998)); which are produced by sintering cancellous tissue of animals (JP Patent Publication (Kokai) No. 2000-211978 A); which are produced by treating coral together with phosphoric acid by hydrothermal method so that pores are formed while allowing the skeleton of the coral to be left (Roy DM et al., Nature 247: 220-222 (1974)); which are produced by sintering a slurry of calcium phosphate that contains polyester fiber (Chang BS et al., Biomaterials 21: 1291-1298 (2000)); which are produced by drying a slurry of calcium phosphate step by step to allow pores to communicate with each other in a given direction and the sintering the dried calcium phosphate (JP Patent Publication (Kokai) No. 7-23994 A (1995)); which are not sintered compact, but are produced by binding or attaching calcium phosphate particles with polymer material and gaps among the particles formed and perforations newly and mechanically made are used as pores (JP Patent Publication (Kokai) Nos. 8-276003 A (1996), 11-290447 A (1999)).

### Disclosure of the Invention

Of the above described processes for producing the porous materials of calcium phosphate, artificially synthesized porous materials, other than those produced using calcium phosphate from living organisms, have common problems. Specifically, in artificially synthesized conventional porous materials of calcium phosphate, there is a problem of being unable to increase the ratio of pores which penetrate the porous materials and have a diameter of 70 µm or more while maintaining their strength. In other words, there have been provided no artificially synthesized porous materials of calcium phosphate whose pores all penetrate the porous materials and consist of large pores having a diameter of 70 µm or more and particularly 100 µm or more, whose porosity is 50% or more, and besides whose strength is at a practically sufficient level. Where the pores that penetrate porous materials are 70 µm or less in diameter, where the pores take the form of a dead end and do not penetrate porous materials, or where the pores are closed pores, even if such porous materials are embedded in tissues of living organisms, the invasion and penetration of blood vessels into the porous materials are restricted and thereby the furnishing of nutrition and oxygen is also restricted. This causes insufficient invasion of tissues, such as bone, into the porous materials, resulting in binding of tissues, such as bone, only to the peripheral portions of the porous materials. Furthermore, air having its escape cut off remains in the porous materials, which also contributes to inhibiting the invasion of cells, tissues and blood vessels into the porous materials.

When porous materials that have dead-end and non-penetrating pores or closed pores are used as a cell culture support, phenomenon occurs that the dead-end pores or closed pores are filled with air that has its escape cut off and thereby the porous materials float on culture fluid media and do not sink in the media, or that neither cell culture media nor cells infiltrate into the dead-end pores or closed pores. As a result, the application of such porous materials to the field of tissue engineering or regenerative medicine engineering, which aims at tissue reparation and organ regeneration by in-vitro culturing cells in porous materials of calcium phosphate and returning the cultured cells together with the porous materials to the living bodies, has been restricted.

Low strength of artificially synthesized conventional porous materials is attributed roughly to the following two points. The first point is that since many of conventional porous materials are formed not by compression press molding, but by the procedure of drying slurries of powder, the adhesion among powder particles results insufficient and the particle adhesion after sintering is poor, whereby the strength is not increased. The second point is that since the size and the arrangement of pores in conventional porous materials are disorderly, when compressive load is applied, shearing force acts on anywhere in pore walls or beams that form the porous structure, whereby the beams and the walls are fractured.

On the other hand, porous materials which have neither end-shaped pores nor closed pores, whose pores are all penetrating themselves and 70 µm or more in diameter, from which air is promptly expelled when they are in a liquid, and which allow good invasion and penetration of blood vessels into themselves, and thus which are applicable to tissue engineering or regenerative medicine engineering are practically limited to those of calcium phosphate from living organisms. The porous materials of calcium phosphate from living organisms use bones and corals as raw materials. And in the porous structure of the tissues of these living organisms, the size of pores and the arrangement of beams are orderly so that it undergoes not shear but buckling alone when compressive load is applied thereto. As a result, porous materials of calcium phosphate from living organisms have high strength, despite the fact that all their pores consist substantially of those penetrating themselves and 70 µm or more in diameter. The porous materials of calcium phosphate from living organisms thus have many excellent points; but on the other hand, they are at a disadvantage in that their chemical compositions and phase compositions cannot be selected freely and their resorption and properties of facilitating tissue regeneration cannot be controlled.

Accordingly the object of this invention is to provide a porous material of calcium phosphate with high strength which has strength equal to or higher than that of porous materials of calcium phosphate from living organisms; whose pores all penetrate itself and consist of large pores 70 µm or more and preferably 100 µm or more in size so that it allows air to be expelled from itself when it is in a liquid and blood vessels to invade and perforate itself or cells to infiltrate into itself; whose porosity is at a sufficient level; whose chemical composition can be freely changed so that Ca/P molar ratio varies within the range of 0.75 to 2.1; to which elements important for facilitating osteogenesis activity and producing resorption can be added; and whose phase composition can also be changed relatively easily.

In this invention, artificially formed, three-dimensional and penetrated open pores mean those which are formed one by one using long columnar bodies as male dies, which have directional properties of penetrating a sintered compact in two or more directions, whose beginning and end positions are intentionally designed, which penetrate through the sintered compact, and whose spacing and arrangement are artificially designed.

In this invention, to artificially form three-dimensional penetrate open pores, a large number of long columnar bodies having a cross-sectional size of 90 µm or more and 5.0 mm or less and preferably 100 µm or more and 3.0 mm or less and having a length of 3-fold or more and preferably 10-fold or more the cross-sectional size are used as male dies for forming pores. The materials for long columnar male dies are one kind or more than one kind of solid selected from the group consisting of: metals; woods; bamboo or other plant materials; woods; carbon materials; halogen-free polymers having a modulus of elasticity of 10 GPa or more, such as polyethylene, nylon, polyacetal, polycarbonate, polypropylene, polyester, ABS, polystyrene, phenol, urea resin, epoxy resin and acrylate; and preferably halogen-free thermosetting polymers having a modulus of elasticity of 10 GPa or more, such as polyester, phenol resin, urea resin and epoxy resin. The reason for the use of these kinds of solid is that they have a high modulus of elasticity. Specifically, in this invention, since the long columnar male dies are pressurized at 5 MPa or more and 500 MPa or less and preferably 10 MPa or more and 200 MPa or less during the forming operation, if they have a modulus of elasticity of 10 GPa or less, they themselves undergo a deformation of 0.05% or more, which in turn causes fracture of the compact due to the pore closing during the pressurizing or due to the form restoration of the long columnar bodies after the pressurizing. If halogen-containing polymers are used, the halogen reacts with calcium phosphate during the sintering to produce chlorine apatite (Ca₁₀(PO₄)₆Cl₂) or fluorine apatite (Ca₁₀(PO₄)₆F₂), which are poor in biocompatibility. Use of thermosetting polymers makes it possible to avoid the reaction of the polymers with the powder or binder used which is caused by their melting and thereby decreases closing of pores during the firing.

The volume fraction of the long columnar bodies for forming penetrated open pores to the compact obtained after compression molding is 20% or more and 90% or less and preferably 30% or more and 80% or less. If the volume fraction of the long columnar bodies to the compact after the compression molding is less than 20%, a sufficient amount of cells and blood vessels are not allowed to be introduced into the pores of the compact after sintering and the porous material obtained from the compact is therefore practically of little value. If the volume fraction of the long columnar bodies to the compact after the compression molding is more than 90%, the porous material obtained has markedly decreased strength and is not suitable for practical use.

The shape of the cross section of the long columnar bodies is not limited to any specific one; however, a polygon having at least one pair of sides parallel to each other, an oval, a circle, or a figure formed of at least one pair of sides parallel to each other and curves is advantageous in compression molding and drawing the long columnar bodies out of the compact. The shape across the length of the long columnar bodies needs to be a rectilinear figure without a curve or a curvilinear or broken-line-like figure with curves on one plane alone. If it is a curvilinear figure with curves on two or more planes, it interferes with compression molding, because a deformation is caused in the long columnar male dies during the compression molding, which in turn causes a fracture in the long columnar male dies as well as a fracture in the compact due to the form restoration of the long columnar bodies after pressurizing.

The size of the cross section of the long columnar male dies depends on the pore size finally required. In artificial bones or porous materials of calcium phosphate used for tissue engineering, pore size is needed after sintering which allow at least more than one vascular endothelial cell or osteoblast 30 µm in size to invade one pore at a time. To allow the pore size after sintering to be 70 µm or more, the cross-sectional size of the long columnar male dies needs to be 90 µm or more, because pores shrink by 10 to 20% by sintering. In artificial bones or porous materials of calcium phosphate used for tissue engineering, it is not always necessary to allow blood vessels 4 mm or more in size to invade their pores, and therefore, the cross-sectional size of the long columnar male dies need not be 5.0 mm or more, even taking into consideration 10 to 20% of pore shrinkage. For the reasons described above, the cross-sectional size of the long columnar male dies is 90 µm or more and 5.0 mm or less. The length of the long columnar male dies is 3-fold or more and preferably 10-fold or more their cross-sectional size. If the length of the long columnar male dies is 3-fold or less their cross-sectional size, when powder is added so that all the long columnar bodies used penetrate through the powder, the maximum size of the porous ceramics produced is restricted to about 10 mm, and porous materials having such size are of little value for practical use in artificial bones and tissue engineering.

To enhance the strength of the porous materials to be produced, first these long columnar male dies are arranged, powder is added, and pressure is applied to the plane on which the long columnar male dies are arranged to compress the powder. The long columnar male dies may be arranged parallel to each other at regular intervals, parallel to each other at irregular intervals, or non-parallel to each other as long as they do not overlap. They can be arranged radially so that a plurality of long columnar male dies are concentrated on one spot from its surroundings, multiply radial so that a plurality of long columnar male dies are concentrated on more than one spots from their surroundings, or resinoid. However, when the long columnar male dies are arranged radially, multiply radial or resinoid, it is necessary to bring the end surface portion of one long columnar male die completely into contact with the end surface portions of the other long columnar male dies by fitting, bonding, etc. The portion which is incompletely in contact with the other portions contributes to the formation of a pore having a dead end after sintering. The pressure applied during compression molding is 5 MPa or more and 500 MPa or less and preferably 10 MPa or more and 200 MPa or less. The reason for setting the pressure during compression molding in the above range is that if the pressure is 5 MPa or less, the adhesion among powder particles results insufficient, which makes it impossible to produce a porous ceramic having sufficient strength, whereas if the pressure is 500 MPa or more, the long columnar male dies are more likely to deform or fracture. Further, if the pressure is 500 MPa or more, when intending to draw the long columnar male dies out of the pressurized compact after the completion of stacking operation, the male dies cannot sometime be drawn out or they can sometimes be worn due to the friction produced between the powder and themselves. This is problematic when long columnar metal male dies are used.

In this invention, precursors of calcium phosphate mean calcium phosphate which becomes sintered compact of calcium phosphate after sintering and the above calcium phosphate which contains at least one selected from the group consisting of carbonic acid, silicon, magnesium, zinc, iron and manganese.

In this invention, "calcium phosphate in which elements or carbonic acid is dissolved" means: when one or more than one metal element such as magnesium, zinc, iron or manganese are dissolved in calcium phosphate, calcium phosphate in which part of calcium is substituted with one or more than one of the above elements as impurities; when silicon is dissolved in calcium phosphate, calcium phosphate in which part of phosphorous is substituted with silicon as an impurity; and when carbonic acid is dissolved in calcium phosphate, calcium phosphate in which part of phosphoric acid is substituted with carbonic acid as an impurity. When silicon or carbonic acid is dissolved in calcium phosphate, there is discrepancy between the charge of the atom or the ion group to be replaced and that of silicon or carbonic acid; as a result, secondary dissolution of other elements occurs or vacant sites where no atoms exist are produced in the structure so as to compensate the discrepancy. For example, when carbonic acid is dissolved in hydroxyapatite Ca₁₀(PO₄)₆(OH)₂, simultaneous substitution of (Na⁺, CO₃²⁻) for (Ca²⁺, PO₄³⁻) or of (H⁺, CO₃²⁻) for (Ca²⁺, PO₄³⁻) occurs. Each element or ion group has a solubility limitation. As a result, when intending to dissolve an element such as silicon, magnesium, zinc, iron or manganese in calcium phosphate beyond the solubility limit, the oxide or phosphate of such an element is formed, besides calcium phosphate in which the element is dissolved, and thus a composition is provided which contains the oxide or phosphate. In low-temperature-type Ca₃(PO₄)₂, the solubility limits of magnesium, zinc, iron and manganese are all about 12 mol% of the total amount of calcium.

As powders of calcium phosphate precursors, those whose Ca/P molar ratio is 0.75 or more and 2.1 or less and preferably 1.1 or more and 1.9 or less can be used. Even if the Ca/P molar ratio is 1.5 or less, in calcium phosphate precursors that contain an impurity selected from the group consisting of carbonic acid, silicon, magnesium, zinc, iron and manganese, for example, in calcium phosphate precursors that contain magnesium, a mixture of magnesium dissolved tricalcium phosphate and trimagnesium phosphate is formed and thus the formation of calcium pyrophosphate, which is poor in biocompatibility, can be prevented. However, if the Ca/P molar ratio is 0.75 or less, though addition of an impurity selected from the group consisting of carbonic acid, silicon, magnesium, zinc, iron and manganese enables the formation of calcium pyrophosphate to be prevented, the mole number of such an impurity becomes larger than that of calcium and thereby resultant sintered compact is not that of calcium phosphate. Thus the minimum of the Ca/P molar ratio of the calcium phosphate precursor powders used is 0.75. If the Ca/P molar ratio is 2.1 or more, calcium oxide is formed in amounts beyond its toxic limit, and the biocompatibility of resultant porous materials after sintering deteriorates. Thus the maximum of the Ca/P molar ratio of the calcium phosphate precursor powders used is 2.1. The particle size of the calcium phosphate precursor powders used is not limited to any specific one; however, preferably it is in the range of about 0.1 µm to 100 µm.

In calcium phosphate precursors that contain none of carbonic acid, silicon, magnesium, zinc, iron and manganese, preferably the Ca/P molar ratio is 1.5 or more and 2.0 or less. Concrete examples of such calcium phosphate precursors are: hydroxyapatite; tricalcium phosphate; tetracalcium phosphate; amorphous calcium phosphate; each of which independently has a Ca/P molar ratio of 1.5 or more and 2.0 or less, and the mixtures thereof; and besides, the powders of each of the above described compounds and mixtures with which powder having a Ca/P molar ratio of 1.5 or more and 2.0 or less, for example, a calcium salt such as calcium hydrogenphosphate, calcium glycerophosphate, metal calcium, calcium oxide, calcium carbonate, calcium lactate, calcium citrate, calcium nitrate or calcium alkoxide, ammonium phosphate, or phosphoric acid is mixed. These compounds may have a stoichiometric or non-stoichiometric composition.

Concrete examples of calcium phosphate precursors that contain carbonic acid are: carbonic-acid-dissolved hydroxyapatite; carbonic-acid-dissolved amorphous calcium phosphate; the mixture thereof; and calcium phosphate precursors to which sodium carbonate, potassium carbonate or ammonium carbonate is added.

Concrete examples of calcium phosphate precursors that contain silicic acid are: silicic-acid-dissolved hydroxyapatite; silicic-acid-dissolved amorphous calcium phosphate; silicic-acid-dissolved tricalcium phosphate; the mixtures thereof; and calcium phosphate precursors to which calcium silicate or silicic acid is added.

Concrete examples of calcium phosphate precursors that contain magnesium, zinc, iron, or manganese are: hydroxyapatite, amorphous calcium phosphate, tetracalcium phosphate and tricalcium phosphate in which metal ions as above are dissolved; and calcium phosphate precursors to which one or more than one of the above metals, or the metal oxides, hydroxides, phosphates, nitrates or carbonates thereof is added. The chlorides, fluorides and sulfates of the metals cannot be used because they allow chlorine, fluorine and sulfuric group, which are poor in biocompatibility, to remain during sintering.

In this invention, "binder" means organic or inorganic substances having bonding properties which are added to calcium phosphate precursors so that the processes of forming and sintering the precursor powder are done well. Concrete examples of such binders are polyvinyl alcohol and carboxymethyl cellulose.

In this invention, "solvent" means substances that are added to calcium phosphate precursors so that the flowability and adhesion of the precursors are improved. Concrete examples of such solvents are water, alcohols, and other volatile organic solvents.

The amount of calcium phosphate precursor powder added needs to be weighed out so that it is 103% or more and less than 114% and preferably 104% or more and 106.5% or less of the amount of calcium phosphate powder calculated from the equation (volume of the clearance among long columnar bodies) x (theoretical value of calcium phosphate density). If the amount is 103% or less, the powder is hard to pressurize. If the amount is 114% or more, the column surface of the long columnar bodies is completely buried in the powder and does not come in contact with the adjacent long columnar bodies during the stacking process described below. In this case, after pressurizing the powder, excess powder is removed from each of the long columnar bodies so that the powder and the long columnar bodies are at the same level.

If the powder is added in amounts within the preferable range, that is, 104% or more and 106.5% or less, part of the surface of each long columnar bodies is exposed, which makes it possible to form continuous pores extending at right angles with the plane on which the long columnar bodies are oriented. However, even when the powder is added in amounts within the preferable range, 104% or more and 106.5% or less, it is better to carry out the step of removing excess powder from each of the long columnar bodies so that the powder and the columnar bodies are at the same level, because the step allows much more pores to continuously extend at right angles with the plane on which the long columnar bodies are oriented.

As a binder added to the calcium phosphate precursor powder, polyvinyl alcohol can be used and its amount is 2 wt% or more and 10 wt% or less and preferably 2 wt% or more and 5 wt% or less, just like the case of ordinary compression molding of calcium phosphate. If the amount of polyvinyl alcohol added is 10 wt%, the walls or beams of the porous materials after sintering become porous, which means insufficient improvement in strength. If the amount of polyvinyl alcohol added is 2 wt% or less, the adhesion among the powder particles is poor and thereby compression molding is impossible. Preferably the amount of solvent added is 5 wt% or more and 52 wt% or less and preferably 10 wt% or more and 45 wt% or less. Addition of a solvent improves the flowability of the powder and thereby the clearance among the long columnar male dies can be filled with the powder during the pressurizing of the powder. When a solvent is added, a drying step for evaporating the solvent is carried out before the sintering step. The reason for setting the amount of solvent added at 5% or more and 52% or less is that if the amount is 5% or less, the solvent-added powder is practically the same as a dry powder and the effect of adding a solvent cannot be produced, whereas if the amount is 52% or more, the walls or beams of the porous materials after sintering become porous, which means insufficient improvement in strength.

A plurality of single-layer compression molded products are stacked which are produced using a calcium phosphate precursor, or a composition made up of a calcium phosphate precursor and a binder, or a composition made up of a calcium phosphate precursor, a binder and a solvent depending on the situation. The stacking process may be carried out in such a manner as to prepare a plurality of single-layer molded products in advance and stack them at a time or in such a manner as to compression mold one single-layer product and mold another single-layer product on the above single-layer product. In the stacking process, a plurality of single-layer products are stacked so that each of the long columnar male dies in one single-layer product comes in contact with vertically adjacent long columnar male dies at more than one point and the long columnar male dies in one single-layer product extend in the direction different from that in which the long columnar male dies in adjacent single-layer products do. Thus, the contact points among the long columnar male dies form continuous pores extending in the die-stacked direction. The pressure applied during the compression molding is 5 MPa or more and 500 MPa or less and preferably 10 MPa or more and 200 MPa, just like the case of the single-layer forming process.

To enhance the functions of living bodies, one kind or more than one kind of element, which is selected from the group consisting of zinc, magnesium, iron, manganese and silicon, essential to living bodies can be added to the calcium phosphate precursors. The content of zinc, iron, manganese or silicon in the porous ceramics after sintering should be in the range of 1-fold to 100-fold the content of the same in bone. The contents of zinc, iron, manganese and silicon in bone are as follows. Zinc: 0.012 wt% to 0.0217 wt%, iron: 0.014 wt% to 0.02 wt%, manganese: 1 ppm to 4 ppm, and silicon: 0.0105 wt%. If the content such an element in porous materials is less than 1-fold the content of the element in bone, the effect of facilitating the function of living bodies specific to the element cannot be produced. If the content of such an element in porous materials is more than 100-fold the content of the element in bone, the element exists in excess both in bone tissue and in tissue engineering scaffold used in a cell culture medium and develops toxicity. If the content of such an element in porous materials is 25-fold or more and 100-fold or less the content of the element in bone, the element develops toxicity in bone tissue, but not in a cell culture medium and thus the porous materials cab used as a tissue engineering scaffold. The content of magnesium in porous materials after sintering should be in the range of 1-fold to 50-fold the content of magnesium in bone. The content of magnesium in bone is 0.26 wt% to 0.55 wt%. If the content of magnesium in porous materials is 1-fold or less the content of the same in bone, the effect of facilitating the function of living bodies specific to magnesium cannot be produced. The reason for setting the maximum of the magnesium content in porous materials at 50-fold the magnesium content in bone, unlike the other element essential to living bodies, is that the content of magnesium in bone is far large compared with the other elements, and therefore, if the magnesium content in porous materials is 50-fold or more of that in bone, the mole number of magnesium becomes larger than that of calcium in the porous materials after sintering, which means the main component of the porous materials is not calcium phosphate.

The above described elements essential to living bodies may be dissolved in calcium phosphate crystal that makes up the powder of calcium phosphate precursor or may be mixed with the same in the form of an inorganic salt, metal, oxide, hydroxide or organometallic compound. When such inorganic salts, metals, oxides, hydroxides or organometallic compounds are mixed with calcium phosphate crystal in advance, such elements react with calcium phosphate and are dissolved in the same during sintering. The amount of such an element mixed is beyond its solubility limit, besides the element-dissolved calcium phosphate, its metal oxide or phosphate is also produced. When such an element is mixed in the form of an inorganic salt, it is preferable for the element to take the form of a carbonate or nitrate whose negative ion group volatiles during sintering.

To accelerate the disappearance of porous sintered calcium phosphate, which is dissolved and resorbed in living bodies with the passage of time, the powder of calcium phosphate precursor is allowed to contain carbonic acid. The carbonic acid content in the calcium phosphate after sintering is in the range of 0.3 wt% to 5 wt% in terms of CO₃²⁻. The carbonic acid content of 0.3 wt% corresponds to that of bone-like apatite, and with the content equal to or less than 0.3 wt%, calcium phosphate shows more tendency toward precipitating and is unlike to be dissolved/absorbed into living bodies. If the carbonic acid content is 15 wt% or more, it is hard to keep the calcium phosphate phase stable. Since many of carbonates are decomposed at high temperatures and scatter and lose carbonic acid, to allow calcium phosphate precursors to contain carbonic acid, it is preferable to use carbonated apatite obtained by simultaneously substituting the Ca and PO₄ sites of hydroxyapatite Ca₁₀(PO₄)₆(OH)₂ with Na and CO₃ or to add sodium carbonate as an additive.

When the long columnar male dies in the compression molded product formed through the single-layer forming process and the long columnar bodies are formed of metal, they should be drawn out and removed after the layer-stacking process without failure. When the long columnar male dies are formed of other materials such as bamboo, woods, carbon materials, or polymers, they may also be drawn out after the layer-stacking process; however, even if they are kept buried in the powder, they disappear during firing.

When water is added to the powder before compression molding, the resultant compression molded product is dried at room temperature after completing the layer-stacking process until no change is observed in its weight. The drying temperature is not specified, but it is preferable to dry the molded product at room temperature or lower. At drying temperatures of 50°C or higher, when the molded product contains polyvinyl alcohol as a binder, the polyvinyl alcohol degenerates. As a result, the sintered density of the molded product is not increased even by sintering and peeling is more likely to occur at stacking interfaces. At drying temperatures of 40°C or higher, peeling at stacking interfaces can sometimes occur.

The process of sintering the compression molded product is carried out in atmosphere at 500°C or higher and 1500°C or lower and preferably 700°C or higher and 1400°C or lower in an ordinary electric furnace. If the temperature is 500°C or lower, sintering does not occur, whereas if the temperature is 1500°C or higher, much of calcium phosphate is decomposed. The optimal sintering temperature varies depending on the chemical composition of the calcium phosphate powder to be sintered. For example, the optimal sintering temperature of hydroxyapatite containing 3 to 15 wt% of carbonic acid is 600°C or higher and 800°C or lower. The optimal sintering temperature of hydroxyapatite (Ca₁₀(PO₄)₆(OH)₂: Ca/P molar ratio = 1.67) is 900°C or higher and 1200°C or lower. The optimal sintering temperature of hydroxyapatite containing silicon is 900°C or higher and 1200°C or lower. The optimal sintering temperature of low-temperature type tricalcium phosphate (Ca₃(PO₄)₂: Ca/P molar ratio = 1.50) is 900°C or higher and 1100°C or lower. The optimal sintering temperature of low-temperature type tricalcium phosphate containing zinc, manganese or magnesium is 900°C or higher and 1200°C or lower. The optimal sintering temperature of high-temperature type tricalcium phosphate containing zinc, manganese or magnesium is 1300°C or higher and 1500°C or lower.

After completing the sintering process, the density can be measured to determine the porosity. The state where pores are in communication with each other can be assessed by observation under microscope, stereoscope or electron microscope or through staining liquid infiltration. The compressive strength of the porous sintered calcium phosphate can be assessed using Instron type universal tester.

### Brief Description of the Drawings

Figure 1 is a photograph showing the external appearance of a porous sintered compact after firing, whose outside dimensions are 8 mm x 8 mm × 3 mm;
Figure 2 is an electron micrograph showing the pores of a porous sintered compact after firing; and
Figure 3 is a micrograph showing bony tissue formed in the interior of a porous material.

### Best Mode for Carrying Out the Invention

In the following, this invention will be described in more detail by means of examples.

### Example 1

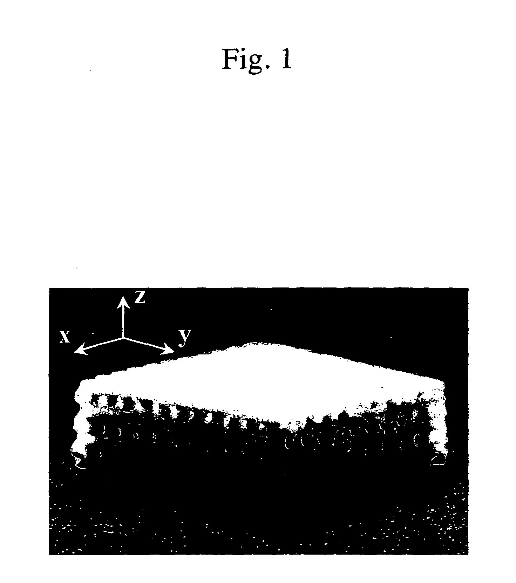

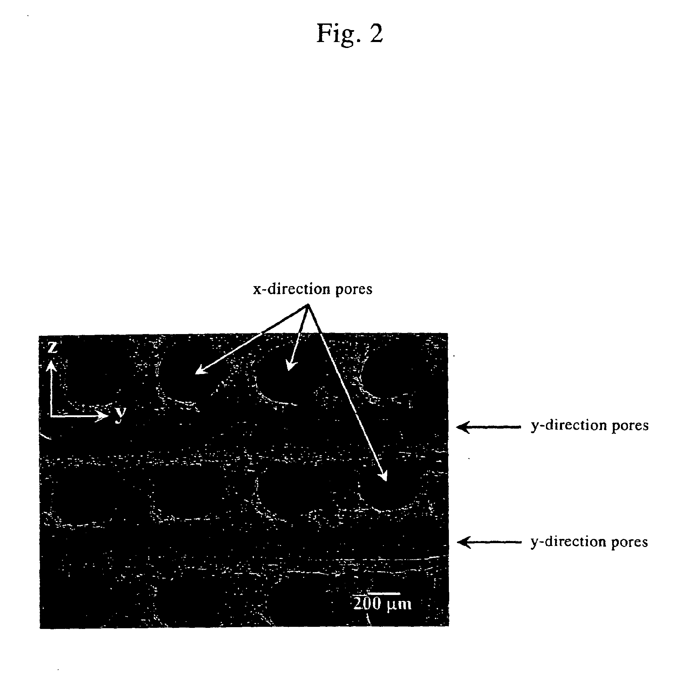

After weighing out 0.175 g of hydroxyapatite powder (Ca₁₀(PO₄)₆(OH)₂: Ca/P molar ratio 1.67) under 75 µm in particle size to which 3% polyvinyl alcohol had been added, 65 microliter of ultra pure water was added to and mixed with the powder. Thirteen long columnar stainless steel male dies 0.5 mm in diameter 28 mm in length were arranged parallel to each other at intervals of 0.3 mm, and 14 long columnar stainless steel male dies of the same size as above were arranged on the above male dies at right angles with the same. The above long columnar male die arrangement was packed with the above powder mixture and pressurized at 36 MPa. After the pressurization, powder that coated the long columnar male dies was removed with a plastic scraper. The above operation was repeated 4 times. After the compression molding, all the long columnar male dies were drawn out to form pores in the compression molded product. The compression molded product was dried for 2 days at room temperature and then sintered for 5 hours at 1170°C to give a porous sintered compact. After the sintering, the porous sintered compact shrank to produce a porous sintered compact in which linear penetrated open pores 380 µm in diameter were spaced at intervals of 200 µm and layers of the linear penetration alternately lay at right angles with each other (refer to Figure 1). The intersections of the linear pores extending in the respective two directions formed pores 50 to 200 µm in diameter; thus, not only the pores as replicas of the long columnar male dies but also continuous pores were formed in the die-stacked direction (refer to Figure 2). Some of the intersections, however, were closed and thus the pores in the die-stacked direction were not necessarily penetrating the porous sintered compact, even though they were open pores. The porosity of the porous sintered compact, as an average of 11 compacts, was 61 ± 3%. This porosity was almost equal to that of the porous apatite obtained by treating coral by hydrothermal method. The SEM observation showed that the structure had fewer pores at its beam portion. A test was performed for the compressive strength of the porous sintered compact in the direction perpendicular to the die-stacked direction, in which the strength of the compact was lowest. The measured result of the compressive strength was 10 MPa or higher, which was equal to or higher than that of the porous apatite from coral.

### Example 2

After weighing out 0.175 g of hydroxyapatite (Ca₁₀(PO₄)₆(OH)₂: Ca/P molar ratio 1.67) powder under 75 µm in particle size to which 3% polyvinyl alcohol had been added, 65 microliter of ultra pure water was added to and mixed with the powder. Thirteen long columnar bamboo or polystyrene male dies 0.5 mm in diameter 30 mm in length were arranged parallel to each other at intervals of 0.3 mm, and 14 long columnar bamboo or polystyrene dies of the same size as above were arranged on the above male dies at right angles. The above long columnar male die arrangement was packed with the above powder mixture and pressurized at 36 MPa. After the pressurization, powder that coated the long columnar male dies was removed with a plastic scraper. The above operation was repeated 4 times. After the compression molding, the compression molded product was dried for 2 days and then sintered for 5 hours at 1170°C to give a porous sintered compact. Both the long columnar bamboo male dies and the long columnar polystyrene male dies were burned down during the sintering process. After the sintering, the porous sintered compact obtained using the long columnar bamboo male dies shrank to produce a porous sintered compact in which linear penetrated open pores 380 µm in diameter were spaced at intervals of 200 µm and layers of the linear penetrated open pores alternately lay at right angles with each other. On the other hand, the porous sintered compact obtained using the long columnar polystyrene male dies partly collapsed because of the distortion of polystyrene during the compression molding operation and the form restoration at an early stage of heating at 200°C or less.

### Example 3

After weighing out 0.175 g of each of different kinds of calcium phosphate precursor powders, different in Ca/P molar ratio, under 75 µm in particle size to which 3% polyvinyl alcohol had been added, 40 to 65 microliter of ultra pure water was added to and mixed with each precursor powder. Thirteen long columnar stainless steel male dies 0.5 mm in diameter 28 mm in length are arranged parallel to each other at intervals of 0.3 mm, and 14 long columnar stainless steel male dies of the same size as above are arranged on the above male dies at right angles with the same. The above long columnar male die arrangement was packed with the above powder mixture and pressurized at 36 MPa. After the pressurization, powder that coated the long columnar male dies was removed with a plastic scraper. The above operation was repeated 4 times. After the compression molding, all the long columnar male dies were drawn out to form pores. The compression molded product was dried for 2 days at room temperature and then sintered for 5 hours at 1100 to 1170°C to give a porous sintered compact. After the sintering, the porous sintered compact shrank to produce a porous sintered compact in which layers of linear penetrated open pores alternately lay at right angles. The intersections of the linear pores extending in the two respective directions formed pores 50 to 200 µm in diameter; thus, not only the pores as replicas of the long columnar male dies but also continuous pores were formed in such a direction that the long columnar male dies were stacked. Some of the intersections, however, were closed. The results are shown in Table 1. When 500 microliter of staining liquid is infiltrated into the resultant porous sintered compacts 8 mm × 8mm × 3 mm in overall size, the staining liquid perforated to the back side of the sintered compact in any direction within 1 to 3 seconds. This indicated that the pores completely penetrated the sintered compact and there was no factor that inhibits the invasion of cells, tissue and blood vessels into the porous sintered compact.

**Table 1**

| Precursor | Ca/P molar ratio | Amount of water added (µl) | Sintering temperature (°C) | Pore diameter (µm) | Porosity (%) |
|---|---|---|---|---|---|
| HAP + TCP | 1.60 | 65 | 1100 | 400 | 64 |
| HAP + TCP | 1.64 | 65 | 1100 | 400 | 65 |
| TCP | 1.50 | 40 | 1100 | 400 | 59 |
| HAP + CC | 1.81 | 45 | 1170 | 380 | 65 |
| HAP: hydroxyapatite Ca₁₀(PO₄)₆(OH)₂ | | | | | |
| TCP: tricalcium phosphate Ca₃(PO₄)₂ | | | | | |
| CC: calcium carbonate CaCO₃ | | | | | |

### Example 4

After weighing out 0.175 g of each of different kinds of calcium phosphate precursor powders under 75 µm in particle size to which 3% polyvinyl alcohol containing elements essential to living bodies had been added, 65 to 80 microliter of ultra pure water was added to and mixed with each precursor powder. Thirteen long columnar stainless steel male dies 0.5 mm in diameter 28 mm in length are arranged parallel to each other at intervals of 0.3 mm, and 14 long columnar stainless steel male dies of the same size as above are arranged on the above male dies at right angles with the same. The above long columnar male die arrangement was packed with the above powder mixture and pressurized at 36 MPa. After the pressurization, powder that coated the long columnar male dies was removed with a plastic scraper. The above operation was repeated 4 times. After the compression molding, all the long columnar male dies were drawn out to form pores. The compression molded product was dried for 2 days at room temperature and then sintered for 5 hours at 1100°C to give a porous sintered compact. After the sintering, the porous sintered compact shrank to produce a porous sintered compact in which layers of linear penetrated open pores alternately lay at right angles. The intersections of the linear pores extending in the two respective directions formed pores 50 to 200 µm in diameter; thus, not only the pores as replicas of the long columnar male dies but also continuous pores were formed in the die-stacked direction. Some of the intersections, however, were closed. The resultant porous sintered compacts were white, but one to which iron was added was light brown. The results are shown in Table 2.

**Table 2**

| Precursor | Ca/P | Metal content (wt %) | Pore diameter (µm) | Porosity (%) |
|---|---|---|---|---|
| HAP + TCP + ZnTCP | 1.61 | 0.84 | 400 | 65 |
| HAP + TCP + ZnTCP | 1.57 | 1.20 | 400 | 62 |
| HAP + TCP + MgTCP | 1.48 | 2.2 | 400 | 76 |
| MgTCP + TMP | 1.12 | 6.1 | 400 | 75 |
| HAP + TCP + Fe | 1.60 | 0.5 | 400 | 64 |
| HAP: hydroxyapatite Ca₁₀(PO₄)₆(OH)₂ | | | | |
| TCP: tricalcium phosphate Ca₃(PO₄)₂ | | | | |
| ZnTCP: 10 mol% zinc-tricalcium phosphate solid solution Ca_{2.7}Zn_{0.3} (PO₄)₂ | | | | |
| MgTCP: 10 mol% Mg-tricalcium phosphate solid solution Ca_{2.7}Mg_{0.3} (PO₄)₂ | | | | |
| TMP: trimagnesium phosphate Mg₃(PO₄)₂ | | | | |
| Fe: iron hydroxide | | | | |

### Example 5

As calcium phosphate precursors, were used carbonated hydroxyapatite powders under 75 µm in particle size which contained 12.5 wt% of carbonate and 7.1 wt% carbonate, respectively. Both kinds of carbonated hydroxyapatite were precipitates obtained by mixing an aqueous solution containing phosphate ions, an aqueous solution containing calcium ions and sodium carbonate. Carbonate group was substituted for part of the phosphate group of hydroxyapatite and sodium was substituted for part of the calcium of hydroxyapatite. After weighing out 0.175 g of each kind of hydroxyapatite powder, 40 microliter of ultra pure water was added to and mixed with the hydroxyapatite. No binder was added. Thirteen long columnar stainless steel male dies 0.5 mm in diameter 28 mm in length are arranged parallel to each other at intervals of 0.3 mm, and 14 long columnar stainless steel male dies of the same size as above are arranged on the above male dies at right angles. The above long columnar male die arrangement was packed with each of the above powder mixture and pressurized at 36 MPa. After the pressurization, powder that coated the long columnar male dies was removed with a plastic scraper. The above operation was repeated 4 times. After the compression molding, all the long columnar male dies were drawn out to form pores. The compression molded product was dried for 2 days and then sintered for 5 hours at 630°C to give a porous sintered compact. The carbonate content after the sintering was decreased by about 6% because part of carbonate group volatilized and scattered due to the sintering. After the sintering, the porous sintered compact shrank to produce a porous sintered compact in which layers of linear penetrated open pores alternately lay at right angles. The intersections of the linear pores extending in the two respective directions formed pores 50 to 200 µm in diameter; thus, not only the pores as replicas of the long columnar male dies but also continuous pores were formed in the die-stacked direction. Some of the intersections, however, were closed. The results are shown in Table 3.

**Table 3**

| Precursor | Ca/P ratio | Carbonate content after sintering (wt %) | Pore diameter (µm) | Porosity (%) |
|---|---|---|---|---|
| CO3AP12 | 1.97 | 6 | 400 | 65 |
| CO3AP7 | 1.81 | 1 | 400 | 62 |
| CO3AP 12: 12.5 wt % carbonated hydroxyapatite solid solution | | | | |
| CO3AP 7: 7.1 wt % carbonated hydroxyapatite solid solution | | | | |

### Example 6

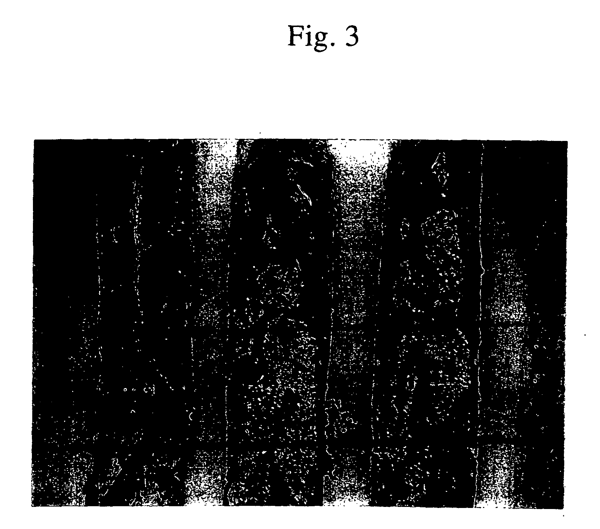

Porous hydroxyapatite having linear penetrated open pores, which was obtained in example 1, and porous hydroxyapatite from coral were dry sterilized for 1 hour at 160°C. The pore diameter and porosity of the porous hydroxyapatite having linear penetrated open pores and the porous hydroxyapatite from coral used are shown in Table 4. Both were almost equal in porosity. Femurs of Fischer 344 strain male rats aged 7 weeks were cut off at their both ends and the marrow cells within the femurs were washed out with 10 mL of cell culture medium. The bone marrow cells taken out of the femurs were cultured for 9 days in Eagle-MEM containing 15% fetal bovine serum, 100 units/mL of penicillin, 100 µg/mL of streptomycin and 0.25 µg/mL of amphotericin B. After the culture, the cells were treated with 0.1% trypsin and cell suspension of 1 × 10⁷/mL was prepared. The above sterilized porous hydroxyapatite having linear penetrated open pores and porous hydroxyapatite from coral were immersed in the cell suspension. Then both kinds of porous hydroxyapatite were implanted into subcutaneous tissue of the dorsa of Fischer 344 strain male rats. Both kinds of implanted porous hydroxyapatite were extracted after 4 weeks and the activity of alkaline phosphatase, an index of osteogenesis activity of osteoblast, was measured as an osteoblast differentiation marker for each kind of porous hydroxyapatite. And the amount of bone Gla-protein, an index of the amount of newly formed bone, was also measured. The measured values of the alkaline phosphatase activity and the amount of bone Gla-protein were each divided by the weight of porous hydroxyapatite. Comparison was made between the resultant values of the porous hydroxyapatite having linear penetrated open pores and the porous hydroxyapatite from coral (n = 4). The results are shown in Table 5 and Table 6. No significant differences in value of the alkaline phosphatase activity were found between the porous hydroxyapatite having linear penetrated open pores and porous hydroxyapatite from coral. No significant differences in amount of bone Gla-protein per unit weight were found, either, between both kinds of porous hydroxyapatite. These indicate that the porous hydroxyapatite having linear penetrated open pores in accordance with this invention has biocompatibility and bioactivity equivalent to those of the porous hydroxyapatite from coral, which has been already used as artificial bone and tissue engineering scaffold, and thus can be used as both artificial bone and tissue engineering scaffold. The extracted porous hydroxyapatite having linear penetrated open pores was fixed, decalsified and cut to thin slices, and the bony tissue formed within the porous hydroxyapatite was stained by hematoxylin-eosin staining and used as decalcified tissue specimens for microscopic observation. The specimens were observed under microscope and whether bony tissue was formed within the porous hydroxyapatite or not was examined (refer to Figure 3). The observation revealed that the porous hydroxyapatite caused no inflammation-related reaction and had high biocompatibility. Further the observation confirmed excellent osteogenisis around the interior of the porous hydroxyapatite and that the porous hydroxyapatite having linear penetrated open pores in accordance with this invention could be used as both artificial bone and tissue engineering scaffold.

**Table 4**

| | Pore diameter (µm) | | | Porosity (%) |
|---|---|---|---|---|
| | x direction | y direction | z direction | |
| Porous ceramic having linear penetrated open pores | 380 | 380 | 50-200 | 61 |
| Porous ceramic from coral | | 190-230 | | 50-65 |

**Table 5**

| | Alkaline phosphatase quantitated per unit weight (micro mol/mg) | Standard deviation |
|---|---|---|
| Porous ceramic having linear penetrated open pores | 0.0333 | 0.0273 |
| Porous ceramic from coral | 0.0338 | 0.0114 |

**Table 6**

| | Amount of bone Gla-protein per unit weight (ng/mg) | Standard deviation |
|---|---|---|
| Porous ceramic having linear penetrated open pores | 1.37 | 0.73 |
| Porous ceramic from coral | 2.84 | 2.82 |

### Industrial Applicability

As described so far, in accordance with this invention, is provided porous material of calcium phosphate of high strength which has strength equivalent to or higher than that of porous material of calcium phosphate from living organisms, whose pores consist of those penetrating itself and having a size of 70 µm or more, whose pores are arranged in a three-dimensional network, whose porosity is sufficiently high for blood vessels to invade and perforate itself or for cells to infiltrate itself, whose chemical composition, in particular, Ca/P molar ratio can be freely changed from 0.75 to 2.1, to which elements important for facilitating osteogenesis and producing resorbable effect can be added, and whose phase composition can be relatively easily changed. Thus the porous material of calcium phosphate in accordance with this invention can be used as artificial bone.

The entire disclosure of the publications cited so far is incorporated in this specification. Those skilled in the art will recognize that various modifications and changes can be made in this invention without departing from the spirit or scope of the following claims. This invention is intended to encompass these modifications and changes.

## Claims

1. Porous sintered compact of calcium phosphate, comprising artificially formed, three-dimensional and perforated open pores from 70 µm to 4 mm in diameter, wherein the porosity is from 20% to 80%, and including calcium phosphate having a Ca/P molar ratio of 0.75 to 2.1 as a main component.

2. The porous sintered compact of calcium phosphate according to claim 1, wherein the calcium phosphate has at least one selected from the group consisting of carbonic acid, silicon, magnesium, zinc, iron and manganese dissolved therein.

3. The porous sintered compact of calcium phosphate according to claim 1, comprising at least one oxide or phosphate of a metal selected from the group consisting of calcium, magnesium, zinc, iron and manganese, in addition to calcium phosphate.

4. The porous sintered compact of calcium phosphate according to claim 2 or 3, wherein the zinc content after sintering is from 0.012 wt% to 1.2 wt%.

5. The porous sintered compact of calcium phosphate according to claim 2, wherein the carbonic acid content after sintering is from 0.3 wt% to 15 wt%.

6. The porous sintered compact of calcium phosphate according to claim 2 or 3, wherein the magnesium content after sintering is from 0.26 wt% to 13 wt%.

7. The porous sintered compact of calcium phosphate according to claim 2 or 3, wherein the silicon content after sintering is from 0.0105 wt% to 1.05 wt%.

8. The porous sintered compact of calcium phosphate according to claim 2 or 3, wherein the iron content after sintering is from 0.014 wt% to 1.4 wt%.

9. The porous sintered compact of calcium phosphate according to claim 2 or 3, wherein the manganese content after sintering is from 1 ppm to 100 ppm by weight.

10. The porous sintered compact of calcium phosphate according to any one of claims 1 to 9, wherein the artificially formed, penetrated open pores from 70µm to 4 mm in diameter arranged in a three-dimensional network.

11. The porous sintered compact of calcium phosphate according to any one of claims 1 to 10, wherein the cross section of the penetrated open pores takes the form of a circle, oval or polygon or has an external form created by a combination thereof.

12. A process for producing porous sintered compact of calcium phosphate, comprising the steps of:
arranging rectilinear long columnar bodies or curvilinear or broken-line-like long columnar bodies with curves on one plane alone on the same plane so that they do not overlap with each other;
arranging additional rectilinear long columnar bodies or curvilinear or broken-line-like long columnar bodies with curves on one plane alone on the plane where the rectilinear long columnar bodies or curvilinear or broken-line-like long columnar bodies with curves on one plane alone have been already arranged so that the additional long columnar bodies do not overlap with each other and extend in the direction different from that in which the long columnar bodies previously arranged extend;
stacking the long columnar bodies arrangements to form a layered structure thereof;
placing a composition comprising a calcium phosphate precursor in the layered structure of the long columnar bodies arrangements so that all the long columnar bodies penetrate each powder of said composition;
compression molding the powder at 5 MPa to 500 MPa so that the long columnar bodies on one plane extend in the direction different from those in which the long columnar bodies on the vertically adjacent two planes extend and come in direct contact with the same; and
sintering the compression molded product in oxidizing atmosphere at 500°C to 1300°C.

13. The process for producing porous sintered compact of calcium phosphate according to claim 12, wherein the composition comprising a calcium phosphate precursor further comprises a binder.

14. The process for producing porous sintered compact of calcium phosphate according to claim 13, wherein in the composition comprising a calcium phosphate precursor and a binder, at least one of the calcium phosphate precursor and the binder is allowed to contain a solvent in advance.

15. The process for producing porous sintered compact of calcium phosphate according to any one of claims 12 to 14, wherein the volume fraction of the long columnar bodies is 20% to 90% of the compression molded product and the compression molded product is sintered in oxidizing atmosphere at 500°C to 1300°C.

16. The process for producing porous sintered compact of calcium phosphate according to any one of claims 12 to 14, wherein the volume fraction of the long columnar bodies is 20% to 90% of the compression molded product and the compression molded product is sintered in oxidizing atmosphere at 500°C to 1300°C after physically or chemically removing the long columnar bodies after molding at 100°C or lower.

17. The process for producing porous sintered compact of calcium phosphate according to any one of claims 12 to 16, wherein the rectilinear long columnar bodies or curvilinear or broken-line-like long columnar bodies with curves on one plane alone whose cross section is any one selected from the group consisting of a circle, an oval and a polygon are made up of one or more materials selected from the group consisting of metals, woods, bamboo or other plant materials, carbon materials and halogen-free polymers having a modulus of elasticity of 10 GPa or more.

18. The process for producing porous sintered compact of calcium phosphate according to any one of claims 12 to 17, wherein the maximum diameter of the rectilinear long columnar bodies or curvilinear or broken-line-like long columnar bodies with curves on one plane alone is 90 µm to 5.0 mm.

19. Artificial bone using the porous sintered compact of calcium phosphate according to any one of claims 1 to 11.

20. A process for producing artificial bone using the process for producing porous sintered compact of calcium phosphate according to any one of claims 12 to 18.

21. A scaffold for tissue engineering using the porous sintered compact of calcium phosphate according to any one of claims 1 to 11.

22. A process for producing a scaffold for tissue engineering using the process for producing porous sintered compact of calcium phosphate according to any one of claims 12 to 18.
